# EUROPÄISCHE PATENTSCHRIFT

(11) **EP 2 783 648 B1**
(45) Veröffentlichungstag und Bekanntmachung des Hinweises auf die Patenterteilung: **23.03.2016**
(21) Anmeldenummer: 14162296.9
(22) Anmeldetag: 28.03.2014
(51) Int. Cl.: A61B 17/72

(54) **Osteosynthesesystem für die multidirektionale, winkelstabile Versorgung von Frakturen von Röhrenknochen umfassend einen Marknagel und Knochenschrauben**
Osteosynthesis system for the multi-directional, angularly stable treatment of fractures of long bones comprising a marrow nail and bone screws
Système d'ostéosynthèse pour l'alimentation multidirectionnelle à angle fixe de fractures d'os longs comprenant un clou médullaire et des vis à os

(30) Priorität: 28.03.2013 DE 102013005413
(43) Veröffentlichungstag der Anmeldung: 01.10.2014
(73) Patentinhaber: Wolter, Dietmar, 22955 Hoisdorf (DE)
(72) Erfinder: Wolter, Dietmar, 22955 Hoisdorf (DE)
(74) Vertreter: Hauck Patentanwaltspartnerschaft mbB

(56) Entgegenhaltungen:
- WO-A1-00/61018
- DE-A1- 19 629 011
- DE-C- 745 873
- DE-U1-202006 015 414

## Beschreibung

Die Erfindung bezieht sich auf ein Marknagel-Osteosynthesesystem für die multidirektionale, winkelstabile Versorgung von Frakturen von Röhrenknochen.

Die Marknagelung ist ein chirurgisches Verfahren zur Versorgung von Brüchen der Röhrenknochen, insbesondere große Röhrenknochen Oberschenkel (Femur), Schienbein (Tibia) und Oberarmknochen (Humerus). Der Marknagel hat einen langgestreckten Nagelkörper aus Metall, der die Fraktur überbrückend in den Markraum des Röhrenknochens eingebracht wird, um die Knochenfragmente während des Heilungsprozesses zu fixieren. Nach der Knochenheilung kann der Marknagel entfernt werden. Vorteilhaft ist die rasche Mobilisierung und Belastbarkeit des Patienten nach der Versorgung.

Die Methode wurde von G. Küntschner entwickelt und vorangetrieben. Während der Zeit des zweiten Weltkrieges wurde sie von ihm klinisch erprobt und hat sich danach weltweit durchgesetzt. Zahlreiche Publikationen über diese Methode und ihre Verbesserungen sind erfolgt. Darüber hinaus ist sie Gegenstand zahlreicher Patentanmeldungen.

Die Marknagelung wird nicht von allen optimistisch betrachtet. Kritikpunkte sind vor allen Dingen eine Beeinträchtigung der Markraumdurchblutung, resultierende Fehlstellungen und schwerwiegende Infektionen.

Küntschner hat zunächst einen Marknagel mit V-förmigem Querschnitt vorgestellt, ferner hat er einen Marknagel in Form eines geschlitzten Metallrohres eingesetzt, um die Verklemmung des Marknagels in dem Knochenrohr zu verbessern. Die vergrößerte Kontaktfläche zwischen diesem Nagel und dem Knochenrohr führte zu einer flächenhafteren Übertragung von Lasten und Kräften. Um diesen Effekt zu verstärken, wurde der Markraum aufgebohrt. Eine zusätzliche Schädigung der Knochendurchblutung war die Folge. Um den Flächenkontakt zwischen Metallrohr und Knochen zu vergrößern, können durch den Knochen hindurch Querschrauben an den Nagelenden angebracht werden. Hierfür weisen die Marknägel an den Enden kreisförmige bzw. schlitzförmige Löcher auf, durch welche die Verriegelungsschrauben hindurchgeführt werden.

Weit verbreitet sind Marknägel, die einen zentralen Durchgangskanal aufweisen. Zum Setzen des Marknagels wird ein Führungsdraht oder Führungsspieß in den Markraum eingeführt. Der Marknagel wird über das Führungselement in den Markraum eingeschlagen. Hierfür wird ein Einschlagelement mit einem verbreiterten Kopf auf das obere Ende des Marknagels geschraubt. Mit einem Hammer wird der Marknagel eingetrieben. Das Führungselement stellt sicher, dass der Marknagel nach dem Austritt aus dem einen Frakturteil in das andere Frakturteil eintritt.

Um die Gefäßschädigung durch das Aufbohren zu reduzieren, wurden in den vergangenen Jahrzehnten auch die sog. unaufgebohrten Marknägel angewandt. Hierbei handelt es sich um massive Marknägel, die in der Regel keinen Hohlraum aufweisen. Sie bestehen aus solidem Material und bieten auch die Möglichkeit zur Verriegelung.

Marknägel sind anatomisch vorgeformt. Ferner sind sie in verschiedenen Dicken und Längen erhältlich. Sie werden vorzugsweise aus einem Edelstahl oder einer Titanlegierung hergestellt.

Die gattungsbildende DE 43 41 677 C1 beschreibt einen Verriegelungsnagel für die Versorgung von Knochenfrakturen mit einem länglichen Körper aus Vollmaterial, der am vorderen, distalen Ende abgerundet ist und ein proximales Einschlagende aufweist. Im Verlauf des länglichen Körpers sind Querbohrungen zur Aufnahme jeweils einer Knochenschraube angeordnet. Jede Querbohrung weist zumindest an einer Seite jeder Querbohrung eine trichterförmige, sich nach außen erweiternde Öffnung auf. Die trichterförmigen Öffnungen dienen dazu, einen ungenau am Knochen angesetzten und die Querbohrungen nicht genau treffenden Bohrer problemlos durch die jeweilige Querbohrung hindurch zu führen. Durch eine Vielzahl von voneinander beabstandeten Querbohrungen soll eine größere Flexibilität bei der Fixierung eines Verriegelungsnagels, beispielsweise bei der Versorgung einer Trümmerfraktur sichergestellt werden. Durch seinen geringen Querschnitt soll der Verriegelungsnagel die Gefahr von Durchblutungsstörungen mindern. Der bekannte Verriegelungsnagel wird durch die vielen Querbohrungen mit den trichterförmigen Öffnungen geschwächt. Die Schwächung ist insbesondere in der Mitte des Marknagels gegeben, die den höchsten Belastungen unterworfen ist. Deshalb besteht die Gefahr, dass der Marknagel unter Belastung bricht.

Die DE 196 29 011 A1 beschreibt einen Marknagel, der für jede Verriegelungsschraube ein Durchgangsloch hat, das zu den beiden Seiten hin trichterförmige Aufweitungen und in der Mitte einen Gewindebereich hat. Die Übergänge zwischen den Aufweitungen und dem Gewindebereich sind gerundet. Die Durchgangslöcher sind in Inlays ausgebildet, die an ihrem zylindrischen Außenumfang mit dem Grundmaterial des Marknagels verschweißt sind. Das Grundmaterial und die Verriegelungsschrauben bestehen aus einem Titan mit einem höheren Härtgrad und das Inlay aus einem Titan mit einem geringeren Härtegrad. Infolgedessen sind die Verriegelungsschrauben unter unterschiedlichen Winkeln (multidirektional) unter Formung eines Gewindes sowohl in eine Verbohrung des Knochens auf beiden Seiten des Marknagels als auch in die Durchgangslöcher einschraubbar. Hierdurch wird eine multidirektionale, winkelstabile Verriegelung ermöglicht. Der verringerte Marknagelquerschnitt an den trichterförmig aufgeweiteten Durchgangslöchern schwächt den Marknagel. Nach diesem Prinzip wurden Marknägel hergestellt, die an den Marknagelenden derartig multidirektional verblockbare Schrauben aufweisen. An den Marknagelenden sind die verringerten Querschnitte unkritisch, weil der Marknagel dort nur verhältnismäßig geringen Belastungen unterworfen wird.

Das Dokument DE202006015414U U1 offenbart ein Osteosynthesesystem gemäß dem Oberbegriff von Anspruch 1.

Die EP 1 143 867 B1 beschreibt einen Marknagel, der auf beiden Seiten der Fraktur mindestens ein Durchgangsloch aufweist. Das Durchgangsloch hat auf beiden Seiten sphärisch nach außen sich erweiternde Bereiche und in der Mitte einen gewindetragenden Bereich. Der gesamte Marknagel kann aus einem verhältnismäßig harten Titan bestehen. In Durchgangslöcher beidseitig der Fraktur sind Verriegelungsbolzen eingesetzt. Ein Verriegelungsbolzen ist konisch und hat an den beiden Enden Gewinde zum Einschrauben in den Knochen, die aus einem härteren Titanmaterial bestehen. Dazwischen hat er einen konischen Mantelbereich, der aus einem weicheren Titanmaterial besteht. Beim Einschrauben des Verriegelungsbolzens durch eine Vorbohrung des Knochens in ein Durchgangsloch formt der Verriegelungsbolzen auf beiden Seiten des Marknagels ein Gewinde in den Knochen ein und formt das Gewinde des Durchgangslochs ein Gewinde in den Mantelbereich des Verriegelungsbolzens ein. Der Verriegelungsbolzen ist in verschiedenen Winkelausrichtungen winkelstabil mit dem Marknagel verschraubbar. Nachteilig bei diesem Marknagel ist die Querschnittsschwächung im Bereich der Durchgangslöcher, die unter Belastung zum Versagen führen kann. Zudem hat sich herausgestellt, dass eine Gewindeformung durch Eindrehen eines weichen Bolzenteils in ein hartes Muttergewinde ungünstig ist.

Trotz der beschriebenen Vorteile ist auch bei der Marknagelung der Heilungsprozess langwierig. Während des Heilungsprozesses ist der Knochen nur eingeschränkt belastbar.

Davon ausgehend liegt der Erfindung die Aufgabe zugrunde, ein Osteosynthesesystem mit einem Marknagel und Knochenschrauben zur Verfügung zu stellen, das den Heilungsprozess wirksamer unterstützt und die Belastbarkeit des Röhrenknochens während des Heilungsprozesses erhöht.

Die Aufgabe wird durch ein Osteosynthesesystem mit den Merkmalen von Anspruch 1 gelöst. Vorteilhafte Ausgestaltungen des Osteosynthesesystems sind in Unteransprüchen angegeben.

Das erfindungsgemäße Osteosynthesesystem für die Versorgung von Frakturen von Röhrenknochen umfasst einen Marknagel mit einem langgestreckten Nagelkörper aus einem harten Metallmaterial mit zwei Endabschnitten und einem Mittelabschnitt dazwischen. Der Nagelkörper weist mindestens ein quergerichtetes endnahes Schraubenloch zum Einschrauben mindestens einer Knochenschraube in jedem Endabschnitt auf. Der Nagelkörper ist massiv oder weist einen zentral im Querschnitt des Nagelkörpers angeordneten Durchgangskanal zum Durchschieben eines Führungsdrahtes, Führungsspießes oder anderen Führungselements auf. Zudem weist der Nagelkörper im Mittelabschnitt mindestens zwei in Längsrichtung des langgestreckten Nagelkörpers voneinander beabstandete, quergerichtete einseitige Schraubenlöcher zum Einschrauben von Knochenschrauben in der Nähe einer Fraktur auf. Sämtliche einseitigen Schraubenlöcher sind in Inlays (Einsatzelementen) aus einem weichen Metallmaterial ausgebildet, das mit dem langgestreckten Nagelkörper fest verbunden ist. Ferner umfasst das Osteosynthesesystem Knochenschrauben mit einem Gewindeanfang aus einem harten Metallmaterial, die unter Einformung von Gewinden in den Knochen und die Schraubenlöcher einschraubbar sind, wodurch sie den Knochen fest mit dem Marknagel verbinden.

Dem erfindungsgemäßen Osteosynthesesystem liegt die Erkenntnis zugrunde, dass die bekannte Verriegelung der Marknagelenden in dem aufgrund seiner Schwammstruktur weichen Knochen der Epiphyse Mikrobewegungen der Frakturenden des Knochens nicht entscheidend reduziert.

Die Reduzierung der Mikrobewegungen, und zwar vor allem bei Biegung, Scherung und Torsion, ist jedoch eine Voraussetzung für eine ungestörte Heilung.

Die Erfindung schafft hier Abhilfe, indem sie eine frakturnahe winkelstabile Verblockung des Marknagels durch Einschrauben von Knochenschrauben in den kortikalen Knochen der Diaphyse und in die einseitigen Schraubenlöcher ermöglicht. Der kortikale, diaphysäre Knochen weist aufgrund seiner kompakten Knochensubstanz eine erhöhte mechanische Festigkeit auf. Trotz der Schwächung durch die einseitigen Schraubenlöcher kann der besonders hohen Belastungen ausgesetzte Mittelabschnitt des Marknagels hinreichend stabil ausgeführt werden.

Dies beruht zunächst darauf, dass die einseitigen Schraubenlöcher jeweils nur von einer Längsseite des Nagelkörpers ausgehen und sich nicht bis zur gegenüberliegenden Längsseite des Nagelkörpers erstrecken, sondern in einem Abstand von der gegenüberliegenden Längsseite enden. Die einseitigen Schraubenlöcher können als Sacklöcher ausgebildet sein. Bei einem aufgebohrten Marknagel sind sie vorzugsweise jeweils nur auf einer Seite des Durchgangskanals angeordnet. Weiterhin vorzugsweise münden sie innen im Durchgangskanal des Nagelkörpers. Infolgedessen ist der Querschnitt des Nagelkörpers durch die einseitigen Schraubenlöcher weniger geschwächt als durch Schraubenlöcher, die sich quer durch den gesamten Querschnitt hindurch erstrecken, wie bei den herkömmlichen Marknägeln.

Die Festigkeit des Knochens und des Marknagels ist durch diese unikortikale Verankerung des Marknagels weniger beeinträchtigt. Für eine feste Verankerung des Marknagels im Knochen ist dies hinreichend, weil das Material des Marknagels (z.B. ein festes Reintitan oder eine Titanlegierung) eine höhere Festigkeit als das Knochenmaterial aufweist. Durch Einschrauben einer Knochenschraube ist ein von einer Längsseite des Nagelkörpers ausgehendes Gewinde in frei wählbaren Winkelausrichtungen (multidirektional) in das Inlay aus weichem Material (z.B. ein weiches Reintitan) einformbar. Zudem benötigt der erfindungsgemäße im Unterschied zum herkömmlichen Marknagel keine Schraubenlöcher mit trichterförmigen Erweiterungen, um eine Knochenschraube multidirektional in ein Inlay im Inneren des Marknagels einzuschrauben. Eine starke Schwächung des Marknagelquerschnitts durch trichterförmig erweiterte Schraubenlöcher kann vermieden werden.

Das Auffinden der einseitigen Schraubenlöcher kann dadurch erschwert werden, dass sich der Marknagel beim Einschlagen in den Röhrenknochen verformt. Deshalb ist nicht gewährleistet, dass mittels eines vorübergehend am Marknagelende befestigten Setzgeräts ein Bohrer zum Vorbohren eines Loches im Knochen genau auf die einseitigen Schraubenlöcher ausgerichtet werden kann. Das Vorbohren von Knochenlöchern und Setzen der Knochenschrauben kann unter Zuhilfenahme eines Röntgengeräts zur Ermittlung der Position der einseitigen Schraubenlöcher erfolgen. Dadurch, dass die Knochenschrauben multidirektional in den Marknagel einschraubbar sind, wird das Auffinden der einseitigen Schraubenlöcher erleichtert.

Das Osteosynthesesystem ist vorteilhaft durch Modifikation herkömmlicher Osteosynthesesysteme mit aufgebohrtem oder nicht aufgebohrtem Marknagel herstellbar. Herkömmliche Systemkomponenten und zugehörige Werkzeuge können vorteilhaft weiterverwendet werden. Alternativ wird ein erfindungsgemäßes Osteosynthesesystem völlig neu hergestellt.

Der Marknagel ist vorzugsweise anatomisch vorgeformt. Vorzugsweise ist z.B. bei einem Marknagel für den Unterschenkelknochen (Tibia) ein Endabschnitt gegenüber dem Mittelabschnitt leicht abgewinkelt. Vorzugsweise ist der Endbschnitt mit dem der Marknagel voran in den Knochen eingetrieben wird, zu seinem Ende hin verjüngt.

Der Marknagel kann verschiedene Querschnittsformen aufweisen. Beispielsweise kann er einen kreisrunden Querschnitt aufweisen und einen dreieckigen Querschnitt (ggf. mit Radien an den drei "Ecken") oder einen kleeblattförmigen Querschnitt. Ferner kann der Marknagel außen über den Umfang verteilte Nuten und/oder Rippen aufweisen.

Der Nagelkörper kann ein solider bzw. massiver Nagelkörper ohne Durchgangskanal sein, d.h. ein Nagelkörper mit einem Vollquerschnitt. Ferner kann der Nagelkörper ein solider bzw. massiver Nagelkörper mit einem gebohrten Durchgangskanal sein. Alternativ ist der Nagelkörper durch Umformen eines Bleches zu einem Rohr mit kreisrundem, ovalem, eckigem oder anderem Querschnitt hergestellt. Vorzugsweise wird ein Blechstreifen durch Biegen, Abkanten oder Wickeln (Wickelfalzen) zu einem Rohr umgeformt.

Gemäß einer Ausgestaltung ist der Marknagel geschlitzt. Der Schlitz kann über der gesamten Länge des Nagelkörpers erstreckt sein, oder aber nur über den mittleren Bereich. Bei einem Nagelkörper aus einem Blechstreifen wird der Schlitz von den längsseitigen Rändern des Blechstreifens begrenzt. Gemäß einer anderen Ausgestaltung ist der Nagelkörper an den längsseitigen Rändern des Blechstreifens durch eine Schweißnaht geschlossen.

Die quergerichteten endnahen und einseitigen Schraubenlöcher sind jeweils in einem Winkel zur Längsachse des Marknagels am jeweiligen Ort des Schraubenloches ausgerichtet. Vorzugsweise sind sie in einem rechten Winkel zur Längsachse ausgerichtet. Sie können aber auch in einem spitzen Winkel zur Längsachse ausgerichtet sein. Ferner können verschiedene Schraubenlöcher unter verschiedenen Winkeln zur Längsachse ausgerichtet sein.

Die multidirektionale, winkelstabile Fixierung von Knochenschrauben in Knochenplatten, Marknägeln und anderen Verbindungsträgern sind in der DE 43 43 117 C2 und in der EP 1 143 867 B1 beschrieben. Gemäß vorteilhaften Ausgestaltungen der Erfindung sind die Fixierungen der mittleren Knochenschrauben in den mittleren Schraubenlöchern so ausgestaltet, wie in den beiden Dokumenten beschrieben, deren Inhalt hiermit in die vorliegende Patentanmeldung aufgenommen wird.

Der Nagelkörper und die Gewindeanfänge der Knochenschrauben bestehen jeweils aus einem harten Metallmaterial und die Inlays aus einem weichen Metallmaterial.

Das bedeutet, dass das Metallmaterial der Inlays weicher ist, als das Metallmaterial des Nagelkörpers und das Metallmaterial der Gewindeanfänge der Knochenschrauben. Ferner können der Nagelkörper und die Gewindeanfänge der Knochenschrauben aus gleich harten Metallmaterialien oder aus verschieden harten Metallmaterialien bestehen.

Der Gewindeanfang der Knochenschrauben ist das Gewindeprofil bzw. der Anfang des Gewindeprofils der Knochenschrauben, das bzw. der ein Gewinde in die Schraubenlöcher des Marknagels einformt. Beispielsweise besteht nur eine äußere, das Gewindeprofil oder einen Anfang des Gewindeprofils umfassende Schicht der Knochenschrauben oder nur der den Gewindeanfang aufweisende Teil des Schraubenbolzens aus dem harten Metallmaterial und bestehen die Knochenschrauben im Übrigen aus einem weichen Metallmaterial. Vorzugsweise bestehen die Knochenschrauben jeweils vollständig aus dem harten Metallmaterial.

Der Nagelkörper und die Gewindeanfänge der Knochenschrauben bestehen vorzugsweise aus einer Titanlegierung oder einem harten Reintitan oder einem anderen harten Titanmaterial. Die Titanlegierung umfasst vorzugsweise Titan, Vanadium und Aluminium. Weiterhin umfasst sie vorzugsweise ca. 95% Titan und insgesamt ca. 5% Vanadium und Aluminium. Weiterhin vorzugsweise wird eine Titanlegierung Grade 5 oder höher oder Reintitan Grade 4 oder 3 für den Nagelkörper und/oder die Knochenschrauben verwendet.

Die Inlays bestehen vorzugsweise aus einem weichen Reintitan. Weiterhin vorzugsweise wird für die Inlays Reintitan Grade 0, 1 oder 2 verwendet. Die Inlays können aus einem anderen Material als Reintitan bestehen, welches eine geringere Härte als das Material des Nagelkörpers und als das Material der mittleren Knochenschrauben aufweist, z.B. aus einer Titan-Niob-Legierung.

Anstatt eines Titanmaterials können für den Nagelkörper und/oder die Knochenschrauben auch andere Materialien verwendet werden, die eine größere Härte als das Material der Inlays aufweisen. Der Nagelkörper und die Knochenschrauben können aus den gleichen oder aus verschiedenen Materialien bestehen.

Gemäß einer Ausgestaltung sind die Inlays in quergerichtete einseitige Löcher im Nagelkörper eingesetzt. Die einseitigen Löcher erstrecken sich von einer Längsseite des Nagelkörpers aus in den Nagelkörper hinein und enden in diesem in einem Abstand von der gegenüberliegenden Längsseite. Die einseitigen Löcher können als Sacklöcher ausgebildet sein. Bei einem aufgebohrten Marknagel münden sie vorzugsweise in einem Durchgangskanal des Nagelkörpers. Gemäß einer weiteren Ausgestaltung münden die einseitigen Löcher im Durchgangskanal und ragen die Inlays nicht in den Durchgangskanal hinein oder versperren nur einen Teil seines Querschnitts.

Alternativ ist der Marknagel ein nicht aufgebohrter Marknagel. Das Inlay (z.B. ein Hohlzylinder) kann ein Durchgangsloch des nicht aufgebohrten Marknagels ausfüllen. Vorzugsweise füllt das Inlay ein Sackloch in einer Längsseite des Nagelkörpers aus.

Das Inlay füllt das einseitige Loch im Nagelkörper vorzugsweise vollständig aus, damit das einseitige Loch den Marknagelquerschnitt möglichst wenig schwächt. Auch können hierdurch Spalte vermieden werden, in denen sich Bakterien ansammeln können. Gemäß einer bevorzugten Ausgestaltung hat das Inlay im einseitigen Loch einen Presssitz. Gemäß einer weiteren Ausgestaltung ist das Inlay an seinem Außenumfang mit dem Innenumfang des Loches und/oder am umlaufenden äußeren Rand mindestens einer Stirnseite mit einem umlaufenden Rand des einseitigen Loches verbunden. Die Verbindung von Inlay und Nagelkörper ist vorzugsweise eine Schweißverbindung. Vorzugsweise ist die Verbindung durch Diffusionsschweißen oder Laserstrahlschweißen hergestellt. Alternativ können die Inlays durch Einschrauben und/oder Nieten in den Löchern des Marknagels befestigt werden.

Bei einem Marknagel mit Durchgangsloch sind gemäß einer Ausgestaltung in einem oder mehreren in Längsrichtung des Nagelkörpers versetzten Querschnitten verschieden ausgerichtete einseitige Schraubenlöcher vorhanden, in die aus verschiedenen Richtungen Knochenschrauben einschraubbar sind.

Gemäß einer weiteren Ausgestaltung hat der Nagelkörper zumindest im Bereich der einseitigen Schraubenlöcher einen verstärkten Querschnitt. Die Verstärkung des Querschnitts ist gemäß einer weiteren Ausgestaltung ein Blechstreifen, der auf der Längsseite des Nagelkörpers gegenüber dem einseitigen Schraubenloch fixiert ist, z.B. durch Anschweißen. Der Blechstreifen besteht vorzugsweise aus demselben Metallmaterial wie der Nagelkörper.

Gemäß einer weiteren Ausgestaltung schließt eine äußere Seitenfläche des Inlays bündig mit der längsseitigen Außenfläche (Mantelfläche) des Nagelkörpers ab und/oder schließt eine innere Seitenfläche des Inlays bündig mit einer den Durchgangskanal begrenzenden längsseitigen Innenfläche des Nagelkörpers ab. Hierdurch wird vermieden, dass das Inlay bezüglich der Außenfläche vorsteht und aufträgt und/oder dass das Inlay das Einführen des Führungselementes behindert. Um den bündigen Abschnitt des Inlays mit dem Nagelkörper zu erreichen, kann ggfs. nach dem Verbinden des Inlays mit dem Nagelkörper eine Nachbearbeitung erfolgen.

Gemäß einer weiteren Ausgestaltung sind die endnahen Schraubenlöcher in mindestens einem Endabschnitt Durchgangslöcher, die sich quer durch den Nagelkörper hindurch erstrecken.

Die Ausführung der endnahen Schraubenlöcher als Durchgangslöcher ist unkritisch, da die Belastung des Nagelkörpers im Bereich der Endabschnitte geringer als im Mittelabschnitt ist.

Ferner ist die Verankerung der Knochenschrauben auf beiden Seiten der Endabschnitte dadurch begünstigt, dass der Röhrenknochen am Knochengelenk voluminöser ist, sodass genügend Knochenmaterial für die Aufnahme von Knochenschrauben zur Verfügung steht, die beidseitig aus dem Marknagel herausstehen. Zudem ist im Bereich der Epiphyse und der Methaphyse eine möglichst großflächige Kraftübertragung von Vorteil, weil sie überhöhte Belastungen des Knochenmaterials vermeidet.

Gemäß einer weiteren Ausgestaltung sind die endnahen Schraubenlöcher in Inlays ausgebildet, die in endnahen Durchgangslöchern des Nagelkörpers angeordnet sind, die sich quer durch den gesamten Querschnitt hindurch erstrecken. Das Auffüllen der endnahen Löcher durch Inlays und die Verbindung des Inlays mit dem Nagelkörper erfolgt vorzugsweise in der oben beschriebenen Weise. Falls die endnahen Löcher einen Durchgangskanal kreuzen, sind gemäß einer Ausgestaltung zwei Einsatzkörper auf verschiedenen Seiten des Durchgangskanals in dem Loch fixiert. Gemäß einer anderen Ausgestaltung sind auch die endnahen Schraubenlöcher einseitige Schraubenlöcher.

Falls mehr als zwei einseitige Schraubenlöcher in Längsrichtung über den Mittelabschnitt verteilt sind, kann der Operateur im jeweiligen Anwendungsfall Knochenschrauben nur in die beiden geeigneten einseitigen Schraubenlöcher einsetzen, die der Fraktur am nächsten sind. Die übrigen einseitigen Schraubenlöcher können unbenutzt bleiben. Hierdurch ist der Marknagel für ganz verschieden gelagerte Frakturen einsetzbar. Der Lageraufwand wird reduziert. Gemäß einer bevorzugten Ausgestaltung sind zwei bis sechs Inlays mit einseitigen Schraubenlöchern in Längsrichtung über dem Mittelabschnitt verteilt.

Gemäß einer weiteren Ausgestaltung sind in mindestens einem Endabschnitt mehrere endnahe Schraubenlöcher jeweils in Längsrichtung und/oder in Umfangsrichtung des Nagelkörpers verteilt angeordnet. Je nach Einsatzfall kann der Operateur geeignete endnahe Schraubenlöcher für das Setzen von Knochenschrauben auswählen. Vorzugsweise sind in mindestens einem Endabschnitt zwei bis drei endnahe Schraubenlöcher vorhanden. Weiterhin vorzugsweise beträgt der Winkelabstand zwischen den benachbarten endnahen Schraubenlöchern jeweils ca. 90°.

Gemäß einer Ausgestaltung sind Füllkörper mit einem Gewinde aus einem harten Metallmaterial und/oder Knochenschrauben unter Einformung von Gewinden zumindest in die einseitigen Schraubenlöcher eingeschraubt. Die Füllkörper (Dummies) bestehen zumindest im Bereich des Gewindeprofils aus einem härteren Metallmaterial als die Inlays. Vorzugsweise bestehen sie insgesamt aus dem harten Metallmaterial. Sie haben bevorzugt denselben Härtegrad wie der Gewindeanfang der Knochenschrauben. Die Füllkörper kompensieren zumindest teilweise die Schwächung des Implantats durch die Schraubenlöcher.

Vorzugsweise sind vor der Operation sämtliche einseitigen Schraubenlöcher durch Dummies besetzt. Entschließt sich der Operateur zu der Methode der frakturnahen Verblockung des Marknagels, kann er vor dem Setzen des Marknagels die Füllkörper aus den Schraubenlöchern entfernen, welche für die frakturnahe multidirektionale winkelstabile Verriegelung sinnvoll erscheinen. Dies kann der Operateur anhand eines Röntgenbildes der Fraktur entscheiden.

Wenn die Knochenschrauben in die frakturnahen Schraubenlöcher eingeschraubt sind, kompensieren diese zumindest teilweise die Schwächung des Implantats durch die frakturnahen Schraubenlöcher. Falls sich der Operateur entschließt, ausschließlich endnahe Schraubenlöcher mit Verriegelungsschrauben zu besetzen, belässt er die Füllkörper in den einseitigen Schraubenlöchern, um die Festigkeit des Marknagels zu erhöhen. Besonders vorteilhaft ist diese Ausgestaltung bei einem Marknagel, der im Mittelabschnitt eine Vielzahl einseitiger Schraubenlöcher aufweist. Der Operateur entfernt vor dem Setzen des Marknagels nur die Füllkörper aus den einseitigen Schraubenlöchern, die der Fraktur am nächsten sind. Die übrigen Füllkörper verbleiben in den einseitigen Schraubenlöchern. Vorzugsweise sind am Ende der Operation alle Nagellöcher entweder durch eine gewindeeinfurchende Schraube oder einen Dummy besetzt.

Das Osteosynthesesystem mit Marknagel, der im Mittelabschnitt eine Vielzahl einseitiger Schraubenlöcher mit Füllkörpern aufweist, ist für eine Vielzahl unterschiedlicher Anwendungen nutzbar. Es müssen nur unterschiedliche Größen und unterschiedliche Ausführungen für verschiedene Röhrenknochen bevorratet werden. Hierdurch wird der Aufwand für die Lagerhaltung reduziert.

Das Osteosynthesesystem kann auch für die Versorgung gelenksnaher Frakturen genutzt werden, insbesondere für Frakturen im Bereich der Metaphyse. Eine frakturnahe Verblockung ist in diesen Anwendungsfällen durch Einschrauben mindestens einer Knochenschraube in ein frakturnahes und endnahes Schraubenloch und mindestens einer Knochenschraube in ein frakturnahes einseitiges Schraubenloch möglich.

Gemäß einer bevorzugten Ausgestaltung steht der Füllkörper innen und/oder außen nicht aus dem Loch heraus. Hierdurch wird vermieden, dass der Füllkörper aufträgt und/oder das Einsetzen eines Führungselementes behindert, falls er vor dem Setzen des Marknagels nicht entfernt wird.

Gemäß einer weiteren Ausgestaltung ist die Unterseite und/oder die Oberseite des Füllkörpers an die Kontur der inneren Seitenflächen und/oder der äußeren Seitenfläche des Nagelkörpers angepasst.

Gemäß einer weiteren Ausgestaltung haben die Knochenschrauben und/oder die Füllkörper einen konischen Gewindebolzen. Durch die konische Form des Gewindebolzens wird die multidirektionale Einschraubbarkeit der mittleren Knochenschrauben in die einseitigen Schraubenlöcher gefördert. Bevorzugt entspricht die Länge der Füllkörper der Länge des einseitigen Schraubenloches. Hierdurch wird ein Auftragen des Gewindebolzens vermieden. Bevorzugt entspricht die Länge des Gewindebolzens der Knochenschrauben der Länge des einseitigen Schraubenlochs zuzüglich der Wandstärke der Kortikalis des Röhrenknochens, für den das Osteosynthesesystem bestimmt ist, und dem Abstand zwischen Marknagel und Kortikalis.

Das Osteosynthesesystem umfasst vorzugsweise Knochenschrauben für einseitige Schraublöcher mit unterschiedlicher Länge und/oder Knochenschrauben für endnahe Schraubenlöcher mit unterschiedlicher Länge. Der Operateur besetzt die Schraubenlöcher jeweils mit Knochenschrauben geeigneter Länge.

Gemäß einer weiteren Ausgestaltung weisen die Knochenschrauben einen scheibenförmigen Schraubenkopf auf. Der scheibenförmige Schraubenkopf trägt auf der Außenseite des Knochens nur wenig auf. Vorzugsweise hat der scheibenförmige Schraubenkopf eine glatte Unterseite, um eine Beschädigung der Knochenoberfläche zu vermeiden.

Gemäß einer weiteren Ausgestaltung weisen die Knochenschrauben und/oder die Füllkörper einen Werkzeugangriff am äußeren Ende auf. Der Werkzeugangriff ist beispielsweise ein Schlitz, Außen-Mehrkant, Innen-Mehrkant, Philipps, Pozidrive, Torks, Innen-Vielzahn, Tri-Wing, Tork-Set, Pentalobe.

Gemäß einer weiteren Ausgestaltung weisen die Inlays am Innenumfang des Schraubenlochs einen vorstehenden umlaufenden Vorsprung oder eine umlaufende Folge von Vorsprüngen auf, in den oder in die durch Einschrauben einer Knochenschraube oder eines Füllkörpers ein Gewinde einformbar ist. Der Vorsprung bildet einen umformbaren Grad, der das Einformen des Gewindes erleichtert und Spanbildung vermeidet. Ausgestaltungen einer multidirektionalen, winkelstabilen Verriegelung mit einem umformbaren Vorsprung sind in der EP1 143 867 B1 beschrieben. In dieser Hinsicht wird Bezug genommen auf die EP1 143 867 B1, deren Inhalt hiermit in die vorliegende Patentanmeldung aufgenommen wird.

Ein aufgebohrter Marknagel mit einem Durchgangskanal im Nagelkörper kann vorteilhaft mittels eines Führungselements gesetzt werden. Das Führungselement wird nach Einbringen des Marknagels in den Röhrenknochen entfernt. Danach werden die Knochenschrauben, deren Spitzen teilweise in den Hohlraum des Marknagels hineinreichen können, eingebracht.

Gemäß einer weiteren Ausgestaltung umfasst das Osteosynthesesystem einen Führungsdraht, Führungsspieß oder anderes Führungselement. Gemäß einer weiteren Ausgestaltung hat das Führungselement einen Querschnitt entsprechend dem Querschnitt des Durchgangskanals oder eines nicht durch die Inlays und/oder die Füllkörper versperrten Teils des Querschnitts des Durchgangskanals, sodass das Führungselement durch den Durchgangskanal hindurchschiebbar ist. Bei einem Führungselement mit Teilkreisquerschnitt können die Verriegelungsschrauben und/oder die Füllkörper in den Durchgangskanal eingreifen. Der Teilkreisquerschnitt ist so gewählt, dass das Führungselement an den Knochenschrauben und/oder Füllkörpern vorbei in dem Durchgangskanal verlagerbar ist.

Die Erfindung wird nachfolgend anhand der anliegenden Zeichnungen von Ausführungsbeispielen näher erläutert. In den Zeichnungen zeigen:

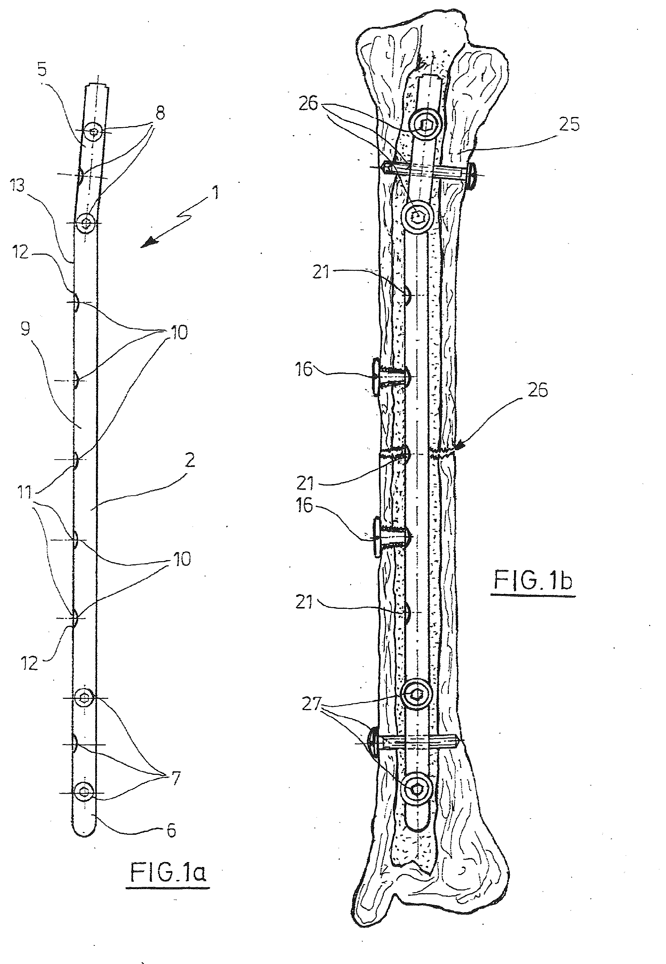

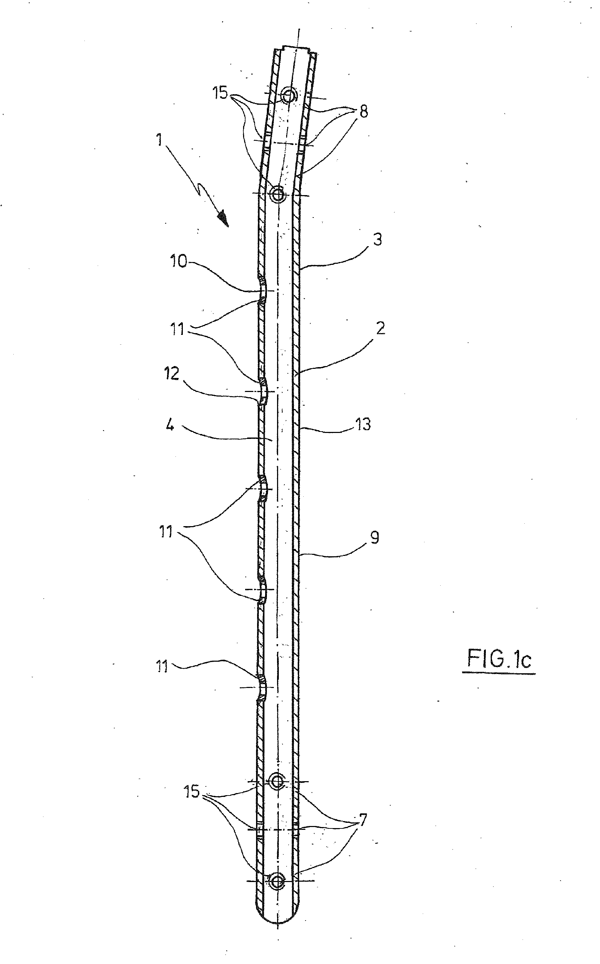

- Fig. 1 a: einen Marknagel in einer Seitenansicht;
- Fig. 1 b: den Marknagel mit Füllkörpern, frakturnahen und endnahen Knochenschrauben in einem Röhrenknochen in einem Längsschnitt durch den Röhrenknochen;
- Fig. 1 c: den Marknagel in einem Längsschnitt
- Fig. 2: den Marknagel in einem Querschnitt durch ein einseitiges Schraubenloch;
- Fig. 3: eine Knochenschraube für ein einseitiges Schraubenloch in einer Seitenansicht;
- Fig. 4: einen Füllkörper in einer Seitenansicht;
- Fig. 5: den Marknagel mit einem Führungsdraht in einem Röhrenknochen in einem Querschnitt;
- Fig. 6: oberer Teil des teilweise längsgeschnittenen Marknagels im Röhrenknochen in einem weiteren Längsschnitt durch den Röhrenknochen;
- Fig. 7: den Marknagel im Röhrenknochen in einem Querschnitt durch einen Füllkörper;
- Fig. 8: den Marknagel im Röhrenknochen in einem Querschnitt durch eine frakturnahe Knochenschraube.

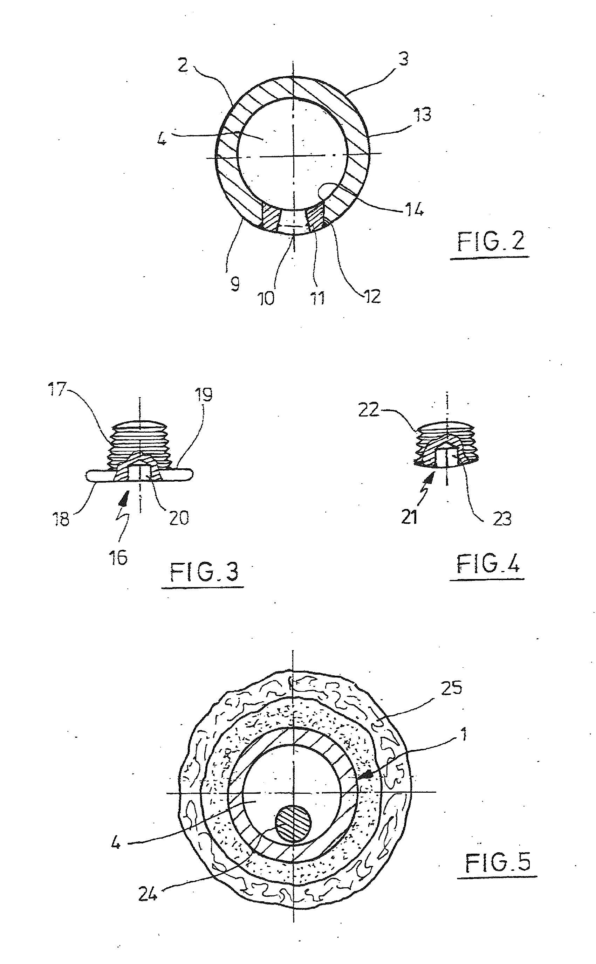

Gemäß Fig. 1 und 2 umfasst ein Marknagel 1 einen langgestreckten Nagelkörper 2 mit einem kreisrunden Querschnitt 3 und einem zentral bezüglich des Querschnittes 3 angeordneten Durchgangskanal 4. Das Lumen des Durchgangskanals 4 ist kreisrund. In einem proximalen Endabschnitt 5 und in einem distalen Endabschnitt 6 des Nagelkörpers 2 sind jeweils drei quergerichtete proximale endnahe Schraubenlöcher 7 und distale endnahe Schraubenlöcher 8 vorhanden. In einem Mittelabschnitt 9 des Körpers 2 sind fünf einseitige Schraubenlöcher 10 vorhanden.

Der langgestreckte Nagelkörper ist aus einer Titanlegierung oder Reintitan Grade 3 oder 4 hergestellt.

Die einseitigen Schraubenlöcher 10 sind jeweils in einem hohlzylindrischen Inlay 11 ausgebildet.

Die Inlays 11 sind aus Reintitan Grade 0, 1 oder 2 hergestellt.

Jedes Inlay 11 ist passgenau in ein kreisrundes einseitiges Loch 12 in der längsseitigen Außenfläche 13 des Nagelkörpers 2 eingesetzt. Die einseitigen Löcher 12 erstrecken sich bis zur längsseitigen Innenfläche 14 des Nagelkörpers 2, die den Durchgangskanal 4 begrenzt. Sie sind jeweils in der dünneren Marknagelwand ausgebildet.

Die Inlays 11 sind mit dem Nagelkörper 2 verschweißt und schließen außen bündig mit der längsseitigen Außenfläche 13 des Nagelkörpers 2 und innen bündig mit der längsseitigen Innenfläche 14 des Nagelkörpers 2 ab. Die einseitigen Schraubenlöcher 10 sind auf nur einer Seite des Durchgangskanals 4 angeordnet. Die Marknagelwand auf einer Seite des Durchgangskanals 4 im Mittelabschnitt 9 des Nagelkörpers 2 weist keine einseitigen Schraubenlöcher 10 auf und ist deshalb ungeschwächt.

Die endnahen Schraubenlöcher 7, 8 erstrecken sich jeweils quer durch den Querschnitt des Nagelkörpers 2 hindurch, d.h. sie weisen Öffnungen in der längsseitigen Außenfläche 13 auf zwei einander gegenüberliegenden Seiten des Nagelkörpers 2 auf. Die endnahen Schraubenlöcher 7, 8 weisen im Beispiel jeweils ein vorgeformtes Innengewinde 15 auf. Alternativ ist jedes endnahe Schraubenloch 7, 8 in einem Inlay 11 ausgebildet, das in einem quergerichteten endnahen Loch mit Nagelkörper 2 angeordnet ist.

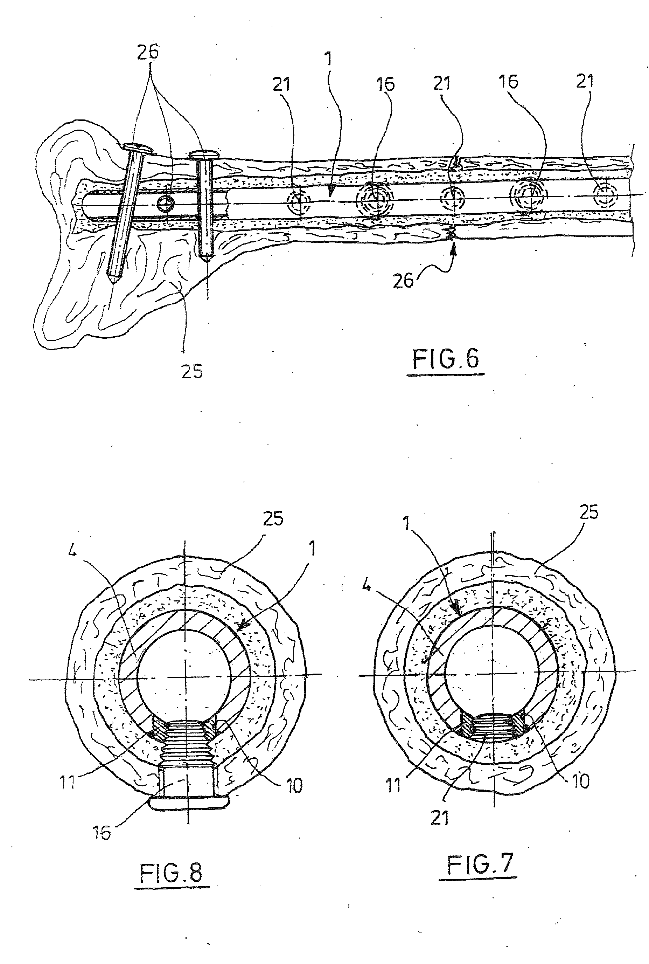

Gemäß Fig. 3 weist eine Knochenschraube 16 für ein einseitiges Schraubenloch 10 einen konischen Gewindebolzen 17 auf und einen scheibenförmigen Schraubenkopf 18. Der maximale Außendurchmesser des Gewindebolzens 17 entspricht etwa seiner Länge. Die Unterseite 19 des Schraubenkopfes 18 ist glatt. Die Oberseite 20 des Schraubenkopfes 18 weist einen Werkzeugangriff auf. Fig. 8 zeigt eine längere Knochenschraube 16.1, deren Gewindebolzen 17 über einen gewindelosen Bolzenabschnitt 17.1 mit dem Schraubenkopf 18 verbunden ist.

Gemäß Fig. 4 besteht ein Füllkörper 21 lediglich aus einem Gewindebolzen 22. Dieser ist so ausgebildet, wie der Gewindebolzen 17 der mittleren Knochenschraube 16. Der Füllkörper 21 weist keinen Schraubenkopf auf. An der Oberseite 23 weist der Gewindebolzen 21 einen Werkzeugangriff auf. Seine Oberseite 23 ist entsprechend der äußeren Seitenfläche des Nagelkörpers gerundet.

Vor dem Setzen des Marknagels 1 sind sämtliche einseitigen Schraubenlöcher 10 mit Füllkörpern 21 besetzt, die unter Einformung von Gewinden in die Inlays 11 eingeschraubt sind. Vorzugsweise sind die Füllkörper 21 senkrecht zur Längsachse des Nagelkörpers 2 in die Inlays 11 eingeschraubt.

Vor dem Setzen des Marknagels 1 entfernt der Operateur die Füllkörper 21 aus den beiden einseitigen Schraubenlöchern 10, zwischen denen nach dem Setzen des Marknagels 1 die Fraktur angeordnet ist.

Gemäß Fig. 5 ist der Marknagel 1 über einen Führungsdraht 24 in einen Röhrenknochen 25 mit der Fraktur 26 einführbar. Die Füllkörper 21 behindern dies nicht, da sie nicht in den Durchgangskanal 4 hineinragen.

Gemäß Fig. 1b und 6 bis 8 sind proximale und distale Knochenschrauben 25, 27 in die proximalen und distalen endnahen Schraubenlöcher 7, 8 eingeschraubt sowie Knochenschrauben 16 in einer wählbaren Winkelausrichtung winkelstabil in die beiden frakturnahen mittleren Schraubenlöcher 10 eingeschraubt. Hierdurch wird zusätzlich zu der proximalen und distalen Verriegelung eine unikortikale frakturnahe Verblockung des Marknagels 1 erreicht.

Sämtliche mittleren Schraubenlöcher 10 sind entweder mit Füllkörpern 21 oder mit Knochenschrauben 16 besetzt, sodass die Schwächung des Querschnitts des Nagelkörpers 4 durch die Schraubenlöcher 10 weitgehend kompensiert wird.

Das Osteosynthesesystem beschleunigt die Heilung der Fraktur und verbessert die Belastbarkeit des Knochens während der Heilung.

## Patentansprüche

1. Osteosynthesesystem für die Versorgung von Frakturen von Röhrenknochen umfassend einen Marknagel (1) mit einem langgestreckten Nagelkörper (2) aus einem harten Metallmaterial mit zwei Endabschnitten (5, 6) und einem Mittelabschnitt (9) dazwischen, der mindestens ein quer gerichtetes endnahes Schraubenloch (7) zum Einschrauben mindestens einer Knochenschraube (25, 27) in jedem Endabschnitt (6) aufweist, wobei
a.) der Nagelkörper (2) massiv ist oder einen zentral im Querschnitt des Nagelkörpers (2) angeordneten Durchgangskanal (4) zum Durchschieben eines Führungselements (24) aufweist,
b.) im Mittelabschnitt (9) mindestens zwei in Längsrichtung des Nagelkörpers (2) voneinander beabstandete, quergerichtete Schraubenlöcher (10) zum Einschrauben von Knochenschrauben (16) in der Nähe einer Fraktur angelegt sind,
c.) alle im Mittelabschnitt angelegten Schraubenlöcher (16) in fest mit dem Nagelkörper (2) verbundenen Inlays (11) aus weichem Metallmaterial ausgebildet sind und wobei
d.) Knochenschrauben (16) mit einem Gewindeanfang aus hartem Metallmaterial unter Einformung von Gewinden in den Knochen und die Schraubenlöcher (10) einschraubbar sind, wodurch sie den Knochen fest mit dem Marknagel verbinden,
**dadurch gekennzeichnet, dass**
die im Mittelabschnitt angelegten Schraubenlöcher einseitige Schraubenlöcher sind, die jeweils nur von einer Längsseite des Nagelkörpers ausgehen und sich nicht bis zur gegenüberliegenden Längsseite des Nagelkörpers erstrecken, sondern in einem Abstand von der gegenüberliegenden Längsseite enden.

2. Osteosynthesesystem nach Anspruch 1, bei dem das Inlay in ein quergerichtetes einseitiges Loch im Nagelkörper (2) eingesetzt ist.

3. Osteosynthesesystem nach Anspruch 1 oder 2, bei dem der Nagelkörper (2) ein massiver Nagelkörper (2) ohne Durchgangskanal (4) oder ein massiver Nagelkörper (2) mit einem gebohrten Durchgangskanal (4) ist.

4. Osteosynthesesystem nach Anspruch 1 oder 2, bei dem der Nagelkörper (2) durch Biegen, Abkanten oder Wickeln eines Blechstreifens hergestellt ist.

5. Osteosynthesesystem nach einem der Ansprüche 3 oder 4, bei dem das einseitige Loch (12) im Durchgangskanal (4) mündet und das Inlay (11) nicht in den Durchgangskanal (4) hineinragt oder nur einen Teil seines Querschnittes versperrt.

6. Osteosynthesesystem nach einem der Ansprüche 1 bis 5, bei dem eine äußere Seitenfläche des Inlays (11) bündig mit einer längsseitigen Außenfläche (13) des Nagelkörpers (2) abschließt und/oder eine innere Seitenfläche des Inlays (11) bündig mit einer den Durchgangskanal (4) begrenzenden längsseitigen Innenfläche (14) des Nagelkörpers (2) abschließt.

7. Osteosynthesesystem nach einem der Ansprüche 1 bis 6, bei dem das Inlay (11) ein Hohlzylinder ist.

8. Osteosynthesesystem nach einem der Ansprüche 1 bis 7, bei dem zwei bis sechs Inlays (11) mit mittleren Schraubenlöchern (10) in Längsrichtung über den Mittelabschnitt (9) verteilt sind.

9. Osteosynthesesystem nach einem der Ansprüche 1 bis8, bei dem Füllkörper (21) mit einem Gewinde aus einem harten Metallmaterial und/oder Knochenschrauben unter Einformung von Gewinden in die mittleren Schraubenlöcher (10) eingeschraubt sind.

10. Osteosynthesesystem nach Anspruch 9, bei dem die Füllkörper (21) außen und/oder innen nicht aus den Schraubenlöchern (10) herausstehen oder innen nur einen Teil des Querschnittes des Durchgangskanals (4) versperren.

11. Osteosynthesesystem nach Anspruch 9 oder 10, bei dem die Füllkörper (21) an einem Ende eine Kehlung aufweisen, die in einer Schraubstellung des Füllkörpers (21) annähernd bündig mit der inneren Seitenfläche (14) des Nagelkörpers (2) abschließt.

12. Osteosynthesesystem nach einem der Ansprüche 3 bis 11, mit einem Führungsdraht oder Führungsspieß (24) mit einem Querschnitt entsprechend dem Querschnitt des Durchgangskanals (4) oder eines nicht durch die Inlays und/oder die Füllkörper versperrten Teils des Querschnittes des Durchgangskanals (4).

13. Osteosynthesesystem nach einem der Ansprüche 1 bis 12, bei dem die mittleren Knochenschrauben (16) einen scheibenförmigen Schraubenkopf (18) haben und/oder die mittleren Knochenschrauben (16) und/oder die Füllkörper (21) einen konischen Gewindebolzen (17, 22) haben.

14. Osteosynthesesystem nach einem der Ansprüche 1 bis 13, bei dem die endnahen Schraubenlöcher (7, 8) Durchgangslöcher sind, die sich quer durch den Nagelkörper (2) hindurch erstrecken.

15. Osteosynthesesystem nach einem der Ansprüche 1 bis 14, bei dem in mindestens einem Endabschnitt (5, 6) mehrere endnahe Schraubenlöcher (7, 8) in Längsrichtung und/oder in Umfangsrichtung des Nagelkörpers (2) verteilt angeordnet sind.

16. Osteosynthesesystem nach einem der Ansprüche 1 bis 15, bei dem das harte Metallmaterial des Nagelkörpers (2) und/oder der Knochenschrauben (25, 27) und/oder der Füllkörper (21) eine Titanlegierung oder ein anderes hartes Titanmaterial und/oder bei dem das weiche Metallmaterial der Inlays ein Reintitan oder ein anderes weiches Titanmaterial ist.

## Claims

1. An osteosynthesis system for treating fractures of tubular bones comprising an intramedullary nail (1) with an elongated nail body (2) consisting of a hard metal material with two end sections (5, 6) and a middle section (9) therebetween that has at least one transverse screw hole (7) close to the end for screwing in at least one bone screw (25, 27) into each end section (6), wherein
a.) the nail body (2) is solid or has a through-channel (4) arranged in the center in the cross-section of the nail body (2) for pushing through a guide element (24),
b.) in the middle section (9), at least two transverse screw holes (10) at a distance from each other in the longitudinal direction of the nail body (2) for screwing in bone screws (16) are placed proximate to a fracture,
c.) all screw holes (16) placed in the middle section are formed as inlays (11) made out of soft metal material securely joined to the nail body (2), and wherein
d.) bone screws (16) with an initial thread consisting of hard metal material can be screwed into the bone and screw holes (10) while forming threads, whereby they securely join the bone and intramedullary nail,
**characterized in that**
the screw holes placed in the middle section are one-sided screw holes which respectively only proceed from one longitudinal side of the nail body and do not extend to the opposite longitudinal side of the nail body, but rather end at a distance from the opposite longitudinal side.

2. The osteosynthesis system according to claim 1, wherein the inlay is inserted into a transverse one-sided hole in the nail body (2).

3. The osteosynthesis system according to claim 1 or 2, wherein the nail body (2) is a solid nail body (2) without a through-channel (4) or a solid nail body (2) with a reamed through-channel (4).

4. The osteosynthesis system according to claim 1 or 2, wherein the nail body (2) can be manufactured by bending, folding or winding a sheet metal strip.

5. The osteosynthesis system according to one of claims 3 or 4, wherein the one-sided hole (12) terminates in the through-channel (4) and the inlay (11) does not extend into the through-channel (4) or only blocks a part of its cross-section.

6. The osteosynthesis system according to one of claims 1 to 5, wherein an outer side surface of the inlay (11) is flush with a longitudinal-side outer surface (13) of the nail body (2) and/or an inner side surface of the inlay (11) is flush with a longitudinal-side inner surface (14) of the nail body (2) bordering the through-channel (4).

7. The osteosynthesis system according to one of claims 1 to 6, wherein the inlay (11) is a hollow cylinder.

8. The osteosynthesis system according to one of claims 1 to 7, wherein two to six inlays (11) with middle screw holes (10) are distributed in the longitudinal direction over the middle section (9).

9. The osteosynthesis system according to one of claims 1 to 8, wherein fill bodies (21) with a thread consisting of a hard metal material and/or bone screws are screwed into the middle screw holes (10) while forming threads.

10. The osteosynthesis system according to claim 9, wherein the fill bodies (21) do not project out of the screw holes (10) to the outside and/or the inside, or only block a part of the cross-section of the through-channel (4).

11. The osteosynthesis system according to claim 9 or 10, wherein the fill bodies (21) have a groove at one end that terminates in a screwed position of the fill body (21) approximately flush with the inner side surface (14) of the nail body (2).

12. The osteosynthesis system according to one of claims 3 to 11, with a guide wire or guide pin (24) with a cross-section corresponding to the cross-section of the through-channel (4) or to a part of the cross-section of the through-channel (4) not blocked by the inlays and/or the fill bodies.

13. The osteosynthesis system according to one of claims 1 to 12, wherein the middle bone screws (16) have a disk-shaped screw head (18), and/or the middle bone screws (16) and/or the fill bodies (21) have a conical threaded pin (17, 22).

14. The osteosynthesis system according to one of claims 1 to 13, wherein the screw holes (7, 8) close to the end are through holes that extend transversely through the nail body (2).

15. The osteosynthesis system according to one of claims 1 to 14, wherein a plurality of screw holes (7, 8) close to the end in at least one end section (5, 6) are arranged distributed in the longitudinal direction and/or the peripheral direction of the nail body (2).

16. The osteosynthesis system according to one of claims 1 to 15, wherein the hard metal material of the nail body (2) and/or of the bone screws (25, 27) and/or of the fill bodies (21) is a titanium alloy or another hard titanium material, and/or wherein the soft metal material of the inlays is a pure titanium or another soft titanium material.

## Revendications

1. Système d'ostéosynthèse pour l'alimentation de fractures d'os longs, comprenant un clou médullaire (1) avec un corps de clou (2) allongé fabriqué en un matériau métallique dur avec deux sections terminales (5, 6) et une section centrale (9) entre les deux, présentant au moins un trou à vis (7) d'orientation transversale proche de l'extrémité pour le vissage d'au moins une vis à os (25, 27) dans chaque section terminale (6), dans lequel
a.) le corps de clou (2) est massif ou présente un canal de passage (4) disposé centralement dans la coupe transversale du corps de clou (2) pour y faire glisser un élément de guidage (24),
b.) dans la section centrale (9), au moins deux trous à vis (10) d'orientation transversale espacés l'un de l'autre dans le sens longitudinal du corps de clou (2) sont réalisés à proximité d'une fracture pour permettre le vissage des vis à os (16),
c.) tous les trous à vis (16) réalisés dans la section centrale sont formés dans des inlays (1) en un matériau métallique souple raccordés de manière fixe au corps de clou (2) et dans lequel
d.) des vis à os (16) avec un début de filet en matériau métallique dur peuvent être vissées en formant des filetages dans les os et les trous à vis (10), ce par quoi elles relient de manière fixe l'os au clou médullaire,
**caractérisé en ce que**
les trous à vis réalisés dans la section centrale sont des trous à vis unilatéraux qui partent respectivement d'un seul côté longitudinal du corps de clou et ne s'étendent pas jusqu'au côté longitudinal opposé du corps de clou, mais aboutissent plutôt à une distance du côté longitudinal opposé.

2. Système d'ostéosynthèse selon la revendication 1, dans lequel l'inlay est inséré dans un trou unilatéral d'orientation transversale dans le corps de clou (2).

3. Système d'ostéosynthèse selon la revendication 1 ou 2, dans lequel le corps de clou (2) est un corps de clou (2) massif sans canal de passage (4) ou un corps de clou (2) massif avec un canal de passage (4) percé.

4. Système d'ostéosynthèse selon la revendication 1 ou 2, dans lequel le corps de clou (2) est fabriqué par cintrage, pliage ou enroulement d'une bande en tôle.

5. Système d'ostéosynthèse selon l'une quelconque des revendications 3 ou 4, dans lequel le trou unilatéral (12) débouche dans le canal de passage (4) et l'inlay (11) ne fait pas saillie dans le canal de passage (4) ou bloque une partie seulement de sa coupe transversale.

6. Système d'ostéosynthèse selon l'une quelconque des revendications 1 à 5, dans lequel une surface latérale extérieure de l'inlay (11) est alignée de façon affleurante sur une surface extérieure (13) côté longitudinal du corps de clou (2) et/ou une surface latérale intérieure de l'inlay (11) est alignée de façon affleurante sur une surface intérieure (14) côté longitudinal du corps de clou (2) limitant le canal de passage (4).

7. Système d'ostéosynthèse selon l'une quelconque des revendications 1 à 6, dans lequel l'inlay (11) est un cylindre creux.

8. Système d'ostéosynthèse selon l'une quelconque des revendications 1 à 7, dans lequel deux à six inlays (11) avec des trous à vis centraux (10) sont répartis dans le sens longitudinal sur la section centrale (9).

9. Système d'ostéosynthèse selon l'une quelconque des revendications 1 à 8, dans lequel des corps de remplissage (21) dotés d'un filetage en un matériau métallique dur et/ou des vis à os sont vissés en formant des filetages dans les trous à vis centraux (10).

10. Système d'ostéosynthèse selon la revendication 9, dans lequel les corps de remplissage (21) ne débordent pas à l'extérieur et/ou à l'intérieur des trous à vis (10) ou bloquent à l'intérieur une partie seulement de la coupe transversale du canal de passage (4).

11. Système d'ostéosynthèse selon la revendication 9 ou 10, dans lequel les corps de remplissage (21) comportent à une extrémité un évidement approximativement aligné de façon affleurante dans une position vissée du corps de remplissage (21) sur la surface latérale intérieure (14) du corps de clou (2).

12. Système d'ostéosynthèse selon l'une quelconque des revendications 3 à 11, avec un fil de guidage ou une broche de guidage (24) avec une coupe transversale correspondant à la coupe transversale du canal de passage (4) ou à une partie non bloquée par les inlays et/ou les corps de remplissage de la coupe transversale du canal de passage (4).

13. Système d'ostéosynthèse selon l'une quelconque des revendications 1 à 12, dans lequel les vis à os centrales (16) ont une tête de vis (18) en forme de disque et/ou les vis à os centrales (16) et/ou les corps de remplissage (21) ont un boulon fileté conique (17, 22).

14. Système d'ostéosynthèse selon l'une quelconque des revendications 1 à 13, dans lequel les trous à vis (7, 8) proches de l'extrémité sont des trous de passage qui s'étendent transversalement à travers le corps de clou (2).

15. Système d'ostéosynthèse selon l'une quelconque des revendications 1 à 14, dans lequel sont répartis dans au moins une section terminale (5, 6) une pluralité de trous à vis (7, 8) proches de l'extrémité dans le sens longitudinal et/ou dans le sens circonférentiel du corps de clou (2).

16. Système d'ostéosynthèse selon l'une quelconque des revendications 1 à 15, dans lequel le matériau métallique dur du corps de clou (2) et/ou des vis à os (25, 27) et/ou des corps de remplissage (21) est un alliage de titane ou un autre matériau en titane dur et/ou dans lequel le matériau métallique souple des inlays est un titane pur ou un autre matériau en titane souple.
